# EUROPÄISCHE PATENTSCHRIFT

(11) **EP 2 782 898 B1**
(45) Veröffentlichungstag und Bekanntmachung des Hinweises auf die Patenterteilung: **21.10.2015**
(21) Anmeldenummer: 12788179.5
(22) Anmeldetag: 13.11.2012
(51) Int. Cl.: C07C 209/16, C07C 211/05, C07C 211/06, C07C 29/145

(54) **VERFAHREN ZUR HERSTELLUNG VON ETHYLAMINEN UND MONO-ISO-PROPYLAMIN (MIPA)**
METHOD FOR PRODUCING ETHYLAMINES AND MONOISOPROPYLAMINE (MIPA)
PROCÉDÉ DE PRÉPARATION D'ÉTHYLAMINES ET DE MONO-ISOPROPYLAMINE (MIPA)

(30) Priorität: 21.11.2011 EP 11189920
(43) Veröffentlichungstag der Anmeldung: 01.10.2014
(73) Patentinhaber: BASF SE, 67056 Ludwigshafen (DE)
(72) Erfinder: ASPRION, Norbert, 67063 Ludwigshafen (DE); JULIUS, Manfred, 67117 Limburgerhof (DE); BEY, Oliver, 67150 Niederkirchen (DE); WERLAND, Stefanie, 68305 Mannheim (DE); STEIN, Frank, 67098 Bad Dürkheim (DE); KUMMER, Matthias, 67098 Bad Dürkheim (DE); MÄGERLEIN, Wolfgang, 68165 Mannheim (DE); MELDER, Johann-Peter, 67459 Böhl-Iggelheim (DE); HUYGHE, Kevin, 2950 Kapellen (BE); MOORS, Maarten, 3670 Meeuwen-Gruitrode (BE)
(86) Internationale Anmeldenummer: PCT/EP2012/072473
(87) Internationale Veröffentlichungsnummer: WO 2013/075974

(56) Entgegenhaltungen:
- EP-A2- 0 284 398
- WO-A1-2007/031449
- US-A1- 2009 234 163
- JUN HEE CHO ET AL: "Reductive amination of 2-propanol to monoisopropylamine over Co/-AlOcatalysts", APPLIED CATALYSIS A: GENERAL, ELSEVIER SCIENCE, AMSTERDAM, NL, Bd. 417, 7. Januar 2012 (2012-01-07), Seiten 313-319, XP028456430, ISSN: 0926-860X, DOI: 10.1016/J.APCATA.2012.01.011 [gefunden am 2012-01-16]

## Beschreibung

Die Erfindung betrifft ein Verfahren zur Herstellung von Ethylaminen und Mono-iso-Propylamin (MIPA).

Mono-iso-Propylamin (MIPA) ist u.a. ein wichtiges Zwischenprodukt zur Herstellung von Pestiziden, Desinfektionsmitteln, Farbstoffen, Weichmachern und Korrosionsinhibitoren und zur Anwendung in der Pharma-Industrie.
Ethylamine eignen sich u. a. als Zwischenprodukte bei der Herstellung von Kraftstoffadditiven, Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

EP 1 106 601 A1 (BASF AG) beschreibt ein Verfahren zur Herstellung von Mono-iso-Propyl-amin aus Aceton und Ammoniak an einem Cu/Ni/Co-Katalysator.

US 7,041,857 B1 (Air Products and Chem. Inc.) lehrt die Hydrierung von Aceton zu Iso-Propanol in flüssiger Phase an einem Schwamm-Metall-Katalysator, besonders Schwamm-Nickel-oder Schwamm-Kobalt-Katalysator, der mit Chrom dotiert ist.

WO 05/063681 A (BASF AG) betrifft ein Verfahren zur Herstellung eines Ethylamins durch Umsetzung von Ethanol mit Ammoniak, einem primären Amin oder einem sekundären Amin in Gegenwart von Wasserstoff und eines Heterogenkatalysators, wobei man ein biochemisch hergestelltes Ethanol (Bio-Ethanol) einsetzt, in welchem zuvor durch In-Kontakt-Bringen mit einem Adsorber Schwefel und/oder schwefelhaltige Verbindungen abgereichert wurden.

WO 06/097468 A (BASF AG) beschreibt ein Verfahren zur Herstellung eines Ethylamins durch Umsetzung von Ethanol mit Ammoniak, einem primären Amin und/oder einem sekundären Amin in Gegenwart von Wasserstoff und eines Heterogen-Hydrier-/Dehydrier-Katalysators, wobei man Bio-Ethanol einsetzt, der Katalysator ein oder mehrere Metalle der Gruppe VIII und/oder IB des Periodensystems enthält und nach Aktivierung mit Wasserstoff eine CO-Aufnahmekapazität von > 100 µmol CO / g Katalysator aufweist.

WO 07/031449 A (BASF AG) lehrt ein katalytisches Verfahren zur Herstellung eines Ethylamins durch Umsetzung von Ethanol mit Ammoniak, einem primären Amin oder einem sekundären Amin in Gegenwart von Wasserstoff wobei man ein Ethanol einsetzt, welches durch Zusatz von Di- und/oder Triethylamin vergällt wurde.

EP 696 572 A1 (BASF AG) betrifft ein Aminierungsverfahren zur Herstellung von Aminen aus primären und sekundären Alkoholen unter Einsatz eines ZrO₂-geträgerten Cu-, Ni- und Mo-haltigen Katalysators. Mögliche Einsatzstoffe sind z.B. Iso-Propanol und Ammoniak.

Eine Alternative zu synthetischem Ethanol ist biologisch oder biochemisch, insbesondere fermentativ hergestelltes, sogenanntes Bio-Ethanol. Dieses wird aus regenerativen Quellen hergestellt und ist damit aus ökologischen Gründen vorteilhaft. Zudem weist Bio-Ethanol teilweise einen günstigeren Preis auf als synthetisches Ethanol.

Beim Einsatz von Bio-Ethanol an vielen Aminierungs-Katalysatoren ist eine deutlich schnellere Katalysatordeaktivierung zu beobachten als dies bei Einsatz von synthetischem Ethanol der Fall ist. Grund dafür sind u.a. der in Bio-Ethanol enthaltene Schwefel und/oder die enthaltenen schwefelhaltigen Verbindungen. Dies wird z.B. in "Fundamentals of Industrial Catalytic Processes", R. J. Farrauto and C. H. Bartholomew, Verlag: Blackie Academic Professional, 1. Auflage, 1997, Seiten 265 - 337, beschrieben; Zitat von Seite 267: "... sulfur- and arseniccontaining compounds are typical poisons for metals in hydrogenation, dehydrogenation and steam reforming reactions". In einer Tabelle auf Seite 268 ist bei speziell Ni, Cu und Co-Katalysatoren Schwefel als eines der gängigen Katalysatorgifte genannt. Die Deaktivierung von Aminierungskatalysatoren mit Schwefel und Schwefelverbindungen thematisieren auch die o.g. Schriften WO 05/063681 A und WO 06/097468 A.

Aufgrund der schnelleren Deaktivierung muss die Synthese häufiger unterbrochen werden, um den Katalysator zu wechseln. Dies führt zu Produktionsausfall, erhöhten Kosten für Katalysator und Katalysatorwechsel und einem erhöhten Personalaufwand verbunden mit erhöhtem Unfallrisiko.

Wird Bio-Ethanol mit einem Gehalt an Schwefel und/oder schwefelhaltigen Verbindungen in Aminierungsverfahren eingesetzt, belegt sich die katalytisch aktive Metalloberfläche des jeweiligen Heterogenkatalysators mit der Zeit mehr und mehr mit dem/den durch den Bio-Alkohol eingetragenen Schwefel bzw. Schwefelverbindungen. Dies führt zu einer beschleunigten Katalysatordeaktivierung und damit zu einer deutlichen Beeinträchtigung der Wirtschaftlichkeit des jeweiligen Prozesses.

Synthetisches Ethanol weist im allgemeinen einen Gehalt an Schwefel und/oder schwefelhaltigen Verbindungen von ≤ 0,1 Gew.-ppm (berechnet S), z.B. bestimmt nach Wickbold (DIN EN 41), auf.

In einer Anlage, in der an demselben Katalysator abwechselnd Bio-Ethanol aminiert und MIPA durch Alkohol-/Ketonaminierung hergestellt wird, tritt demnach das Problem der Katalysatorvergiftung durch einen Gehalt des Bio-Ethanols an Schwefel und/oder schwefelhaltigen Verbindungen auf. Es wird also zwischen beiden Umsetzungen ein Katalysatorwechsel oder Katalysatoraktivierung durch S-Entfernung nötig sein.

Der vorliegenden Erfindung lag die Aufgabe zugrunde, unter Überwindung von Nachteilen des Stands der Technik, ein verbessertes wirtschaftliches Verfahren zur Herstellung von Ethylaminen, d.h. Mono-, Di- und Triethylamin, und Mono-iso-Propylamin (MIPA) bereitzustellen. Das Herstellverfahren sollte sowohl die Ethylamine als auch das MIPA in jeweils in hoher Ausbeute, Raumzeitausbeute (RZA) und Selektivität liefern und zudem besonders einfach und wirtschaftlich sein.

(Raum-Zeit-Ausbeuten werden angegeben in ,Produktmenge / (Katalysatorvolumen • Zeit)' (kg/(IKat. • h)) und/oder ,Produktmenge / (Reaktorvolumen • Zeit)' (kg/(IReaktor • h)).

Erfindungsgemäß wurde erkannt, dass an einem Heterogen-Katalysator, besonders einem kupfer-, nickel- und/oder kobalthaltigen Heterogen-Katalysator, welcher zuvor, z.B. über mehrere Wochen oder Monate, zur Aminierung von Bio-Ethanol eingesetzt und dabei mit Schwefel vergiftet worden war, die nachfolgende Aminierung von Iso-Propanol zu MIPA mit genauso hohen Umsätzen und Selektivitäten gelingt, wie wenn derselbe Katalysator frisch eingesetzt wurde. Mit Aceton als direktem Feed für die MIPA-Herstellung konnte dies hingegen aufgrund Bildung zahlreicher Nebenprodukte nicht erreicht werden. Das Ergebnis ist auch deshalb überraschend, weil die Iso-Propanol-Aminierung mechanistisch eng mit der Aceton-Aminierung verwandt ist, weil die Iso-Propanol-Aminierung über Aceton als Intermediat verläuft (vgl. z.B. E.J. Schwoegler et al., J. Am. Chem. Soc., 1939, Seite 3499-3502).
Vorteilhaft können erfindungsgemäß in einer Anlage in demselben Reaktor und an demselben Katalysator kampagnenweise Ethylamine (EA) aus Bio-Ethanol und MIPA aus Iso-Propanol hergestellt werden, wobei bevorzugt in einem vorgeschalteten Anlagenteil in einem separaten Reaktor Iso-Propanol durch Hydrierung von Aceton hergestellt wird. Die Hydrierung von Aceton wird dabei bevorzugt während der MIPA-Kampagnen durchgeführt und Iso-Propanol, bevorzugt Roh-Iso-Propanol ohne weitere Aufarbeitung, direkt in die Aminierung eingeschleust. Durch dieses Konzept wird ermöglicht, dass die beiden Produkte EA und MIPA in nur einer Anlage hergestellt werden können (Einsparung von Investitionskosten) und gleichzeitig die jeweils am kostengünstigsten verfügbaren Rohstoffe Bio-Ethanol und Aceton (das zunächst zu Iso-Propanol umgesetzt wird) verwendet werden können. Zwischen den beiden Umsetzungen zu EA und MIPA ist kein Katalysatorwechsel nötig und auch keine chemische Katalysatorbehandlung, insb. keine Katalysatoraktivierung durch S-Entfernung (Entgiftung).
Unter einer kampagnenweisen Herstellung von Ethylaminen (EA) und MIPA ist zu verstehen, dass man in derselben Produktionsanlage und am selben Katalysator innerhalb der Lebensdauer des Katalysators in zeitlich begrenzten Abschnitten ("Kampagne") jeweils das eine oder das andere Produkt herstellt. Dabei stellt man während der Lebensdauer des Katalysators mindestens einmal, bevorzugt mindestens zweimal, Ethylamine und mindestens einmal, bevorzugt mindestens zweimal, MIPA her. Beispielsweise können nach Einbau einer frischen Katalysatorcharge drei Monate lang Ethylamine ("Kampagne 1 ") und anschließend vier Monate lang MIPA ("Kampagne 2") produziert werden, anschließend zwei Monate lang wieder Ethylamine ("Kampagne 3") und so weiter, bis das Ende der Lebensdauer des Katalysators erreicht ist. Es kann auch in zwei aufeinanderfolgenden Kampagnen dasselbe Produkt hergestellt werden, z.B. wenn zwischen den beiden Kampagnen eine Anlagenabstellung wegen Inspektion o.ä. liegt.

Demgemäß wurde ein Verfahren zur, insbesondere kampagnenweisen, Herstellung von Ethylaminen und Mono-iso-Propylamin (MIPA) gefunden, welches dadurch gekennzeichnet ist, dass man Bio-Ethanol mit Ammoniak in Gegenwart von Wasserstoff und eines Heterogen-Katalysators zu Ethylaminen umsetzt, wobei das Bio-Ethanol einen Gehalt an Schwefel und/oder schwefelhaltigen Verbindungen von ≥ 0,1 Gew.-ppm (berechnet S) aufweist, und danach in Gegenwart desselben Katalysators Iso-Propanol mit Ammoniak in Gegenwart von Wasserstoff zu MI-PA umsetzt.

### Die Reaktionen erfolgen nach dem Schema

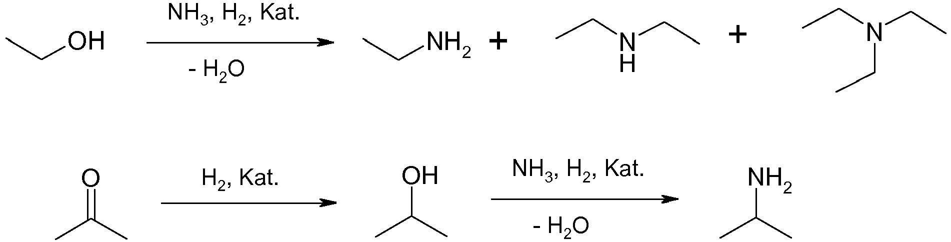

Als Nebenprodukt bei der Aminierung von Iso-Propanol fallen geringe Mengen an Di-(isoPropyl)-amin (DIPA) an.

Bei den Aminierungen mit Ammoniak werden die beiden Alkohole jeweils an demselben (also nicht nur dem gleichen) Heterogen-Katalysator umgesetzt. Es erfolgt also beim Wechsel von Bio-Ethanol zu Iso-Propanol als Reaktorfeed kein Austausch des Katalysators, weder gegen einen Katalysator vom gleichen Typ, noch gegen einen Katalysator von einem anderen Typ. Bevorzugt erhöhter Druck, bevorzugt erhöhte Temperatur und die Anwesenheit von Wasserstoff sind die typischen Reaktionsbedingungen.

Das erfindungsgemäß eingesetzte Bio-Ethanol wird im Allgemeinen aus Agrarprodukten wie Melasse, Rohrzuckersaft, Maisstärke oder aus Produkten der Holzverzuckerung und aus Sulfitablaugen durch Fermentation erzeugt.

Bevorzugt wird Bio-Ethanol eingesetzt, das durch Fermentation von Glukose unter CO₂-Abspaltung erhalten wurde (K. Weissermel und H.-J. Arpe, Industrial Organic Chemistry, WileyVCH, Weinheim, 2003, p. 194; Electronic Version of Sixth Edition of Ullmann's Encyclopedia of Industrial Chemistry, 2000, Chapter Ethanol, Paragraph Fermentation).

Das Bio-Ethanol wird in der Regel durch Destillationsverfahren aus den Fermentationsbrühen gewonnen: Electronic Version of Sixth Edition of Ullmann's Encyclopedia of Industrial Chemistry, 2000, Chapter Ethanol, Paragraph ,Recovery and Purification'.

Insbesondere und vorteilhaft wird im erfindungsgemäßen Verfahren ein biologisch oder biochemisch hergestelltes Ethanol (biologisch oder biochemisch hergestelltes Ethanol = Bio-Ethanol) einsetzt, in welchem nicht zuvor, z.B. durch In-Kontakt-Bringen mit einem Adsorber, wie z.B. Kieselgel, ein aktiviertes Aluminiumoxid, ein Zeolith mit hydrophilen Eigenschaften, eine Aktivkohle oder ein Kohlenstoffmolsieb, Schwefel und/oder schwefelhaltige Verbindungen abgereichert wurden.

Im erfindungsgemäßen Verfahren wird bevorzugt ein Bio-Ethanol eingesetzt, das einen Gehalt an Schwefel und/oder schwefelhaltigen Verbindungen von ≥ 0,1 Gew.-ppm, oder ≥ 0,2 Gew.-ppm, oder ≥ 0,5 Gew.-ppm, oder ≥ 1 Gew.-ppm, oder ≥ 2 Gew.-ppm, oder ≥ 5 Gew.-ppm, oder ≥ 10 Gew.-ppm, (jeweils berechnet S), z.B. bestimmt nach Wickbold (DIN EN 41) (bei S-Gehalten ≤ 2 Gew.-ppm) bzw. coulometrisch bestimmt nach DIN 51400 Teil 7 (bei S-Gehalten > 2 Gew.-ppm), aufweist.
Der Gehalt an Schwefel und/oder schwefelhaltigen Verbindungen kann z.B. bis 200 Gew.-ppm, bis 100 Gew.-ppm, bis 50 Gew.-ppm, bevorzugt bis 10 Gew.-ppm, (jeweils berechnet S), z.B. coulometrisch bestimmt nach DIN 51400 Teil 7, betragen.

Besonders bevorzugt wird ein Bio-Ethanol eingesetzt, das einen Gehalt an Schwefel und/oder schwefelhaltigen Verbindungen im Bereich von ≥ 0,2 bis 2 Gew.-ppm, z.B. ≥ 0,5 bis 2 Gew.-ppm, (jeweils berechnet S), z.B. bestimmt nach Wickbold (DIN EN 41), aufweist.

Bei den schwefelhaltigen Verbindungen kann es sich handeln um anorganische Verbindungen, wie Sulfate, Sulfite, und/oder organische Verbindungen, insbesondere um symmetrische und/oder unsymmetrische C₂₋₁₀-Dialkylsulfide, besonders C₂₋₆-Dialkylsulfide, wie Diethylsulfid, Di-n-propylsulfid, Di-isopropylsulfid, ganz besonders Dimethylsulfid, C₂₋₁₀-Dialkylsulfoxide, wie Dimethylsulfoxid, Diethylsulfoxid, Dipropylsulfoxid, 3-Methylthio-1-propanol und/oder S-haltigen Aminosäuren, wie Methionin und S-Methyl-methionin.

In besonderen Ausführungen wird ein Bio-Ethanol eingesetzt, das zusätzlich zum o.g. Gehalt an Schwefel und/oder schwefelhaltigen Verbindungen
einen Gehalt an C₃₋₄-Alkanolen im Bereich von 1 - 5000 Gew.-ppm, besonders 5 - 3000 Gew.-ppm, ganz besonders 10 - 2000 Gew.-ppm,
einen Gehalt an Methanol im Bereich von 1 - 5000 Gew.-ppm, besonders 5 - 3000 Gew.-ppm, ganz besonders 20 - 1000 Gew.-ppm, und
einen Gehalt an Ethylacetat im Bereich von 1 - 5000 Gew.-ppm, besonders 5 - 3000 Gew.-ppm, ganz besonders 10 - 2000 Gew.-ppm,
aufweist.

Der Gehalt an C₃₋₄-Alkanolen (wie n-Propanol, iso-Propanol, n-Butanol, iso-Butanol, tert.-Butanol), Methanol und Ethylacetat wird z.B. bestimmt mittels Gaschromatographie (30 m DB-WAX-Säule, Innendurchmesser: 0,32 mm, Filmdicke: 0,25 µm, FID-Detektor, Temperaturprogramm: 35 °C (5 Min.), 10 °C/Min. Aufheizrate, 200 °C (8 Min.)).

Die Umsetzung von Bio-Ethanol mit Ammoniak wird bevorzugt über einen Zeitraum durchführt, an dessen Ende der S-Gehalt des Katalysators < 700 Gew.-ppm, besonders < 600 Gew.-ppm, weiter besonders < 500 Gew.-ppm, z.B. 100 bis < 700 Gew.-ppm, besonders 150 bis < 600 Gew.-ppm, beträgt.
Zum Beispiel beträgt dieser Zeitraum beim Einsatz von 0,1 kg Bio-Ethanol / (kg_{Kat}. • h) mit einem Gehalt an Schwefel und/oder schwefelhaltigen Verbindungen von 0,5 Gew.-ppm (berechnet S) bei vollständiger Adsorption des S auf dem Katalysator 4000 Stunden, um einen S-Gehalt des Katalysators von 200 Gew.-ppm zu bewirken.

Der im erfindungsgemäßen Verfahren eingesetzte Katalysator enthält bevorzugt ein oder mehrere Metalle der Gruppe VIII und/oder IB des Periodensystems der Elemente (Chemical Abstracts Service group notation).

Beispiele für solche Metalle sind Cu, Co, Ni und/oder Fe, sowie auch Edelmetalle wie Ru, Pt, Pd, sowie Re. Die Katalysatoren können dotiert sein, etwa mit Ag, Zn, In, Mn, Alkalimetallen (Li, Na, K, Rb, Cs) und/oder Mo.

Der im erfindungsgemäßen Verfahren eingesetzte Heterogen-Katalysator enthält bevorzugt Cu und/oder Ni und/oder Co, bevorzugt Cu und Ni, weiter auch bevorzugt Cu und Ni und Co.

Bevorzugt sind z.B. Heterogen-Katalysatoren, deren Gehalt an Nickel mehr als 90 Gew.-%, besonders mehr als 95 Gew.-%, jeweils bezogen auf alle gegebenenfalls enthaltenen Metalle der Gruppe VIII des Periodensystems (Chemical Abstracts Service group notation), beträgt.

Auch bevorzugt sind z.B. Heterogen-Katalysatoren, deren Gehalt an Kobalt mehr als 90 Gew.-%, besonders mehr als 95 Gew.-%, jeweils bezogen auf alle gegebenenfalls enthaltenen Metal-le der Gruppe VIII des Periodensystems (Chemical Abstracts Service group notation), beträgt.

Beispielsweise sind Raney-Nickel und Raney-Kobalt geeignete Katalysatoren, wobei diese Katalysatoren auch mit weiteren Metallen, wie Cr und/oder Mo und/oder Fe und/oder anderen Metallen der Gruppe VIII des Periodensystems (Chemical Abstracts Service group notation), dotiert sein können.

Für geträgerte Heterogen-Katalysatoren werden als Trägermaterial für die Aktivmetalle bevorzugt Aluminiumoxid (gamma, delta, theta, alpha, kappa, chi oder Mischungen daraus), Siliziumdioxid, Zirkoniumdioxid, Titandioxid, Zeolithe, Alumosilicate, etc. sowie Mischungen aus diesen Trägern verwendet.

Die Katalysatoren können nach bekannten Verfahren, z.B. durch Fällung, Auffällung, Imprägnierung, hergestellt werden.

Bevorzugt enthält der Heterogen-Katalysator ein oxidisches Trägermaterial für die Aktivmetalle, bevorzugt Siliziumdioxid, Aluminiumoxid (gamma, delta, theta, alpha, kappa, chi oder Mischungen daraus), Titandioxid und/oder Zirkoniumdioxid (bevorzugt monokline, tetragonale oder kubische Modifikation). Ein besonders bevorzugtes Trägermaterial ist Aluminiumoxid, insbesondere gamma-Aluminiumoxid.

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.
In diesem Zusammenhang wird das oxidische Trägermaterial, z.B. Aluminiumoxid (Al₂O₃), Zirkoniumdioxid (ZrO₂), als zur katalytisch aktiven Masse gehörig gewertet.

Die Katalysatoren werden dergestalt eingesetzt, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z. B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o. g. Katalysatorträgermaterialien definiert und enthält im wesentlichen die folgenden Bestandteile:
Siliziumdioxid (SiO₂), Aluminiumoxid (Al₂O₃), Titandioxid (TiO₂) und/oder Zirkoniumdioxid (ZrO₂) und sauerstoffhaltige Verbindungen des Kupfers und/oder Nickels und/oder Kobalts.

Die Summe der o. g. Bestandteile der katalytisch aktiven Masse beträgt üblicherweise 70 bis 100 Gew.-%, bevorzugt 80 bis 100 Gew.-%, besonders bevorzugt 90 bis 100 Gew.-%, besonders > 95 Gew.-%, ganz besonders > 98 Gew.-%, insbesondere > 99 Gew.-%, z. B. besonders bevorzugt 100 Gew.-%.
Die katalytisch aktive Masse der erfindungsgemäßen und im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems (Chemical Abstracts Service group notation), enthalten.

### Beispiele für solche Elemente bzw. deren Verbindungen sind:

Übergangsmetalle, wie Mn bzw. MnO₂, Mo bzw. MoO₃, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Erdalkalimetalloxide, wie SrO; Erdalkalimetallcarbonate, wie MgCO₃, CaCO₃ und BaCO₃; Alkalimetalloxide, wie Na₂O, K₂O; Alkalimetallcarbonate, wie Li₂CO₃, Na₂CO₃ und K₂CO₃; Boroxid (B₂O₃).

Bevorzugt enthält die katalytisch aktive Masse des im erfindungsgemäßen Verfahren eingesetzten Katalysators kein Rhenium, kein Ruthenium, kein Eisen und/oder kein Zink, jeweils weder in metallischer (Oxidationsstufe = 0) noch in einer ionischen (Oxidationsstufe ≠ 0), insbesondere oxidierten, Form.

Bevorzugt enthält die katalytisch aktive Masse des im erfindungsgemäßen Verfahren eingesetzten Katalysators kein Silber, weder in metallischer (Oxidationsstufe = 0) noch in einer ionischen (Oxidationsstufe ≠ 0), insbesondere oxidierten, Form.

Bevorzugte Heterogen-Katalysatoren enthalten in ihrer katalytisch aktiven Masse vor der Behandlung mit Wasserstoff
20 bis 90 Gew.-%, bevorzugt 40 bis 85 Gew.-%, besonders bevorzugt 60 bis 80 Gew.-%, sauerstoffhaltige Verbindungen des Aluminiums, berechnet als Al₂O₃,
1 bis 30 Gew.-%, bevorzugt 2 bis 25 Gew.-%, besonders bevorzugt 3 bis 20 Gew.-%, sauerstoffhaltige Verbindungen des Kupfers, berechnet als CuO,
1 bis 40 Gew.-%, bevorzugt 3 bis 30 Gew.-%, besonders bevorzugt 5 bis 20 Gew.-%, sauerstoffhaltige Verbindungen des Nickels, berechnet als NiO, wobei besonders bevorzugt das Molverhältnis von Nickel zu Kupfer größer 1, bevorzugt größer 1,2, besonders bevorzugt 1,8 bis 8,5, ist, und
1 bis 40 Gew.-%, bevorzugt 3 bis 30 Gew.-%, besonders bevorzugt 5 bis 20 Gew.-%, sauerstoffhaltige Verbindungen des Kobalts, berechnet als CoO.

Die sauerstoffhaltigen Verbindungen des Nickels, Kobalts und Kupfers, jeweils berechnet als NiO, CoO und CuO, sind bevorzugt insgesamt in Mengen von 10 bis 80 Gew.-%, besonders bevorzugt 15 bis 60 Gew.-%, ganz besonders bevorzugt 20 bis 40 Gew.-%, in der katalytisch aktiven Masse (vor der Behandlung mit Wasserstoff) enthalten, wobei besonders bevorzugt das Molverhältnis von Nickel zu Kupfer größer 1 ist.

Besonders bevorzugte Heterogen-Katalysatoren enthalten in ihrer katalytisch aktiven Masse vor der Behandlung mit Wasserstoff
20 bis 85 Gew.-%, bevorzugt 20 bis 65 Gew.-%, besonders bevorzugt 22 bis 40 Gew.-%, sauerstoffhaltige Verbindungen des Zirkoniums, berechnet als ZrO₂,
1 bis 30 Gew.-%, besonders bevorzugt 2 bis 25 Gew.-%, sauerstoffhaltige Verbindungen des Kupfers, berechnet als CuO,
14 bis 70 Gew.-%, bevorzugt 15 bis 50 Gew.-%, besonders bevorzugt 21 bis 45 Gew.-%, sauerstoffhaltige Verbindungen des Nickels, berechnet als NiO, wobei bevorzugt das Molverhältnis von Nickel zu Kupfer größer 1, insbesondere größer 1,2, ganz besonders 1,8 bis 8,5, ist, und
0 bis 5 Gew.-%, besonders 0,1 bis 3 Gew.-%, sauerstoffhaltige Verbindungen des Molybdäns, berechnet als MoO₃.

In einer weiteren Variante enthalten diese bevorzugten Katalysatoren zusätzlich in ihrer katalytisch aktiven Masse vor der Behandlung mit Wasserstoff

15 bis 50 Gew.-%, besonders bevorzugt 21 bis 45 Gew.-%, sauerstoffhaltige Verbindungen des Kobalts, berechnet als CoO.

Die sauerstoffhaltigen Verbindungen des Kupfers, Nickels und gegebenenfalls Kobalts, jeweils berechnet als CuO, NiO und CoO, der bevorzugten Katalysatoren sind im allgemeinen insgesamt in Mengen von 15 bis 80 Gew.-%, bevorzugt 35 bis 80 Gew.-%, besonders bevorzugt 60 bis 78 Gew.-%, in der katalytisch aktiven Masse (vor der Behandlung mit Wasserstoff) enthalten, wobei besonders bevorzugt das Molverhältnis von Nickel zu Kupfer größer 1 ist.

### Weitere bevorzugte Heterogenkatalysatoren im erfindungsgemäßen Verfahren sind

in DE 19 53 263 A (BASF AG) offenbarte Katalysatoren enthaltend Kobalt, Nickel und Kupfer und Aluminiumoxid und/oder Siliciumdioxid mit einem Metallgehalt von 5 bis 80 Gew.-%, insbesondere 10 bis 30 Gew.-%, bezogen auf den gesamten Katalysator, wobei die Katalysatoren, berechnet auf den Metallgehalt, 70 bis 95 Gew.-% einer Mischung aus Kobalt und Nickel und 5 bis 30 Gew.-% Kupfer enthalten und wobei das Gewichtsverhältnis von Kobalt zu Nickel 4 : 1 bis 1 : 4, insbesondere 2 : 1 bis 1 : 2, beträgt, beispielsweise der in den dortigen Beispielen verwendete Katalysator mit der Zusammensetzung 10 Gew.-% CoO, 10 Gew.-% NiO und 4 Gew.-% CuO auf Al₂O₃,

in EP 382 049 A (BASF AG) offenbarte bzw. entsprechend herstellbare Katalysatoren, deren katalytisch aktive Masse vor der Behandlung mit Wasserstoff
20 bis 85 Gew.-%, bevorzugt 70 bis 80 Gew.-%, ZrO₂ und/oder Al₂O₃,
1 bis 30 Gew.-%, bevorzugt 1 bis 10 Gew.-%, CuO,
und jeweils 1 bis 40 Gew.-%, bevorzugt 5 bis 20 Gew.-%, CoO und NiO enthält,
beispielsweise die in loc. cit. auf Seite 6 beschriebenen Katalysatoren mit der Zusammensetzung 76 Gew.-% Zr, berechnet als ZrO₂, 4 Gew.-% Cu, berechnet als CuO, 10 Gew.-% Co, berechnet als CoO, und 10 Gew.-% Ni, berechnet als NiO,

in EP 963 975 A (BASF AG) offenbarte Katalysatoren, deren katalytisch aktive Masse vor der Behandlung mit Wasserstoff
22 bis 40 Gew.-% ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, wobei das molare Ni : Cu-Verhältnis größer 1 ist,
15 bis 50 Gew.-% sauerstoffhaltige Verbindungen des Kobalts, berechnet als CoO,
0 bis 10 Gew.-% sauerstoffhaltige Verbindungen des Aluminiums und/oder Mangans, berechnet als Al₂O₃ bzw. MnO₂,
und keine sauerstoffhaltigen Verbindungen des Molybdäns enthält,
beispielsweise der in loc. cit., Seite 17, offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO,

in EP 696 572 A (BASF AG) offenbarte Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂, enthält, beispielsweise der in loc. cit., Seite 8, offenbarte Katalysator mit der Zusammensetzung 31,5 Gew.-% ZrO₂, 50 Gew.-% NiO, 17 Gew.-% CuO und 1,5 Gew.-% MoO₃,

in EP 1 270 543 A1 (BASF AG) beschriebene Katalysatoren, enthaltend mindestens ein Element oder eine Verbindung eines Elementes aus den Gruppen VIII und IB des Periodensystems (Chemical Abstracts Service group notation),

in EP 1 431 273 A (BASF AG) beschriebene Katalysatoren, bei deren Herstellung eine Fällung von katalytisch aktiven Komponenten auf monoklines, tetragonales oder kubisches Zirkoniumdioxid erfolgte,

in EP 636 409 A1 (BASF AG) beschriebene Katalysatoren, siehe insbesondere dort die beispielhaften Katalysatoren A bis E, deren katalytisch aktive Masse aus 55 bis 98 Gew.-% Kobalt, 0,2 bis 15 Gew.-% Phospohor, 0,2 bis 15 Gew.-% Mangan und 0,2 bis 15 Gew.-% Alkalimetall (besonders Natrium), jeweils berechnet als Oxid, besteht, wobei man die Katalysatormasse in einem ersten Schritt bei Endtemperaturen von 550 bis 750 °C und in einem zweiten Schritt bei Endtemperaturen von 800 bis 1000 °C calciniert,
und

Kobalt-Katalysatoren wie sie in US 4,314,084 A (Air Products and Chem., Inc.) beschrieben werden, d.h. umfassend ein Gruppe-VIII-Metall (Chemical Abstracts Service group notation), besonders Kobalt, geträgert auf im Wesentlichen neutralem Aluminiumoxid, wobei der Träger ein Erdalkalimetall, besonders Ca, Ba oder Mg, beinhaltet, insbesondere der in Beispiel 1 genannte Kobalt-Katalysator umfassend ca. 34 Gew.-% Kobalt und einen im Wesentlichen pH-neutralen Al₂O₃-Träger.

Die hergestellten/bezogenen Katalysatoren können als solche gelagert werden. Vor ihrem Einsatz als Katalysatoren im erfindungsgemäßen Verfahren werden sie durch Behandlung mit Wasserstoff vorreduziert (= Aktivierung des Katalysators). Sie können jedoch auch ohne Vorreduktion eingesetzt werden, wobei sie dann unter den Bedingungen des erfindungsgemäßen Verfahrens durch den im Reaktor vorhandenen Wasserstoff reduziert (= aktiviert) werden.

Zur Aktivierung wird der Katalysator bevorzugt bei einer Temperatur im Bereich von 100 bis 500 °C, besonders 150 bis 400 °C, ganz besonders 180 bis 300 °C, über einen Zeitraum von mindestens 25 Min., besonders mindestens 60 Min., einer Wasserstoff-haltigen Atmosphäre oder einer Wasserstoff-Atmosphäre ausgesetzt. Der Zeitraum der Aktivierung des Katalysators kann bis zu 1 h, besonders bis zu 12 h, insbesondere bis zu 24 h, betragen.

Bei dieser Aktivierung wird zumindest ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.
- Herstellung von Ethylaminen aus Bio-Ethanol und Ammoniak:
   Bevorzugt wird die Umsetzung bei einem Absolutdruck im Bereich von 10 bis 100 bar, besonders 15 bis 80 bar, weiter besonders 20 bis 70 bar, durchgeführt.

Die Umsetzung erfolgt bevorzugt bei einer Temperatur im Bereich von 130 bis 230 °C, besonders 150 bis 225 °C, weiter besonders 180 bis 220 °C.

Die Katalysatorbelastung liegt bevorzugt im Bereich von 0,05 bis 0,50 kg/l•h, besonders 0,10 bis 0,35 kg/l•h, weiter besonders 0,15 bis 0,25 kg/l•h [kg Bio-Ethanol / (Liter Katalysator • Stunde)].
(Liter Katalysator = Katalysatorschüttvolumen)

Die eingesetzte Wasserstoffmenge liegt bevorzugt im Bereich von 50 bis 350 Nl/l•h, besonders 100 bis 250 Nl/l•h, weiter besonders 120 bis 200 Nl/l•h, ganz besonders 150 bis 180 Nl/l•h
[Normliter / (Liter Katalysator • Stunde)]
(Nl = Normliter = auf Normalbedingungen (20 °C, 1 bar absolut) umgerechnetes Volumen).

Bevorzugt wird die Umsetzung bei einem Molverhältnis NH₃ : Ethanol im Bereich von 0,4 bis 10 mol/mol, besonders 0,5 bis 5 mol/mol, weiter besonders 0,6 bis 2 mol/mol, durchgeführt.

Das Verfahren lässt sich diskontinuierlich oder bevorzugt kontinuierlich wie folgt durchführen, wobei der Katalysator bevorzugt als Festbett im Reaktor angeordnet ist.

Die Umsetzung wird bevorzugt in einem Rohrreaktor oder Rohrbündelreaktor durchgeführt.

Die Aminierung kann in der Flüssigphase oder in der Gasphase durchgeführt werden. Bevorzugt ist das Festbettverfahren in der Gasphase.

Beim Arbeiten in der Gasphase werden die gasförmigen Edukte (Alkohol plus Ammoniak) in einem zur Verdampfung ausreichend groß gewählten Gasstrom, bevorzugt Wasserstoff, bei den o.g. Drücken und Temperaturen umgesetzt. Es ist dabei sowohl eine Anströmung des Katalysatorfestbetts von oben als auch von unten möglich. Den erforderlichen Gasstrom erhält man bevorzugt durch eine Kreisgasfahrweise.

Sowohl beim Arbeiten in der Flüssigphase als auch beim Arbeiten in der Gasphase ist die Anwendung höherer Temperaturen und höherer Gesamtdrücke möglich. Auch beim Arbeiten in der Flüssigphase ist sowohl eine Anströmung des Katalysatorfestbetts von oben als auch von unten möglich. Der Druck im Reaktionsgefäß, welcher sich aus der Summe der Partialdrücke des Aminierungsmittels, des Alkohols und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe) wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z.B. destillativ.

Aus dem Reaktionsaustrag werden, nachdem dieser zweckmäßigerweise entspannt worden ist, das überschüssige Aminierungsmittel und der Wasserstoff entfernt und die erhaltenen Aminierungsprodukte (Ethylamine) durch Destillation bzw. Rektifikation gereinigt. Das überschüssige Aminierungsmittel und der Wasserstoff werden vorteilhaft wieder in die Reaktionszone zurückgeführt. Das gleiche gilt für eventuell nicht vollständig umgesetzten Bio-Ethanol.
- Herstellung von MIPA aus Iso-Propanol und Ammoniak:
   Bevorzugt wird die Umsetzung bei einem Absolutdruck im Bereich von 10 bis 100 bar, besonders 20 bis 80 bar, weiter besonders 30 bis 60 bar, durchgeführt.

Die Umsetzung erfolgt bevorzugt bei einer Temperatur im Bereich von 130 bis 230 °C, besonders 150 bis 225 °C, weiter besonders 180 bis 220 °C.

Die Katalysatorbelastung liegt bevorzugt im Bereich von 0,05 bis 0,50 kg/l•h, besonders 0,07 bis 0,30 kg/l•h, weiter besonders 0,10 bis 0,25 kg/l•h [kg Iso-Propanol / (Liter Katalysator • Stunde)].
(Liter Katalysator = Katalysatorschüttvolumen)

Die eingesetzte Wasserstoffmenge liegt bevorzugt im Bereich von 50 bis 350 Nl/l•h, besonders 100 bis 250 Nl/l•h, weiter besonders 120 bis 200 Nl/l•h, ganz besonders 150 bis 180 Nl/l•h
[Normliter / (Liter Katalysator • Stunde)]
(Nl = Normliter = auf Normalbedingungen (20 °C, 1 bar absolut) umgerechnetes Volumen).

Bevorzugt wird die Umsetzung bei einem Molverhältnis NH₃ : Iso-Propanol im Bereich von 1,0 bis 10 mol/mol, besonders 1,5 bis 5 mol/mol, weiter besonders 2 bis 4 mol/mol, durchgeführt.

Das Verfahren lässt sich diskontinuierlich oder bevorzugt kontinuierlich wie folgt durchführen, wobei der Katalysator bevorzugt als Festbett im Reaktor angeordnet ist.

Die Umsetzung wird bevorzugt in einem Rohrreaktor oder Rohrbündelreaktor durchgeführt.

Die Aminierung kann in der Flüssigphase oder in der Gasphase durchgeführt werden. Bevorzugt ist das Festbettverfahren in der Gasphase.

Beim Arbeiten in der Gasphase werden die gasförmigen Edukte (Alkohol plus Ammoniak) in einem zur Verdampfung ausreichend groß gewählten Gasstrom, bevorzugt Wasserstoff, bei den o.g. Drücken und Temperaturen umgesetzt. Es ist dabei sowohl eine Anströmung des Katalysatorfestbetts von oben als auch von unten möglich. Den erforderlichen Gasstrom erhält man bevorzugt durch eine Kreisgasfahrweise.

Sowohl beim Arbeiten in der Flüssigphase als auch beim Arbeiten in der Gasphase ist die Anwendung höherer Temperaturen und höherer Gesamtdrücke möglich. Auch beim Arbeiten in der Flüssigphase ist sowohl eine Anströmung des Katalysatorfestbetts von oben als auch von unten möglich. Der Druck im Reaktionsgefäß, welcher sich aus der Summe der Partialdrücke des Aminierungsmittels, des Alkohols und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe) wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z.B. destillativ.

Aus dem Reaktionsaustrag werden, nachdem dieser zweckmäßigerweise entspannt worden ist, das überschüssige Aminierungsmittel und der Wasserstoff entfernt und das erhaltene Aminierungsprodukt (MIPA) durch Destillation bzw. Rektifikation gereinigt. Das überschüssige Aminierungsmittel und der Wasserstoff werden vorteilhaft wieder in die Reaktionszone zurückgeführt. Das gleiche gilt für eventuell nicht vollständig umgesetzten Iso-Propanol.
- Herstellung von Iso-Propanol aus Aceton (Hydrierung):
   Bevorzugter Katalysator ist ein Aluminiumoxid-geträgerter Cu-Katalysator.
Bevorzugt sind weiterhin kupfer- und chromhaltige Katalysatoren (Cu-Cr-Katalysatoren). Weiter bevorzugt ist der in EP 563 327 A = WO 92/10290 A1 (Engelhard Corp.) beschriebene geformte Kupferchromit(III)-Katalysator, hergestellt aus einer Mischung, umfassend 20 bis 80 Gew.-% Kupferchromit(III), worin bevorzugt ein Teil oder das gesamte Kupferchromit(III) die Formel CuO • CuCr₂O₄ hat, und 20 bis 80 Gew.-% von mindestens einem extrudierbaren anorganischen Bindemittelrmaterial, worin der Katalysator eine Oberfläche von 20 bis 225 m²/g hat und das Gesamt-Porenvolumen der Poren mit einem Durchmesser bis zu 9500 Nanometer (95000 Angström) in dem Katalysator 0,35 bis 1 cm³/g beträgt. Siehe insb. dort die beispielhaften Katalysatoren (Beispiele 1-6). Ebenfalls geeignet ist der in WO 92/10290 A1 genannte (Seite 13, Zeile 3), kommerziell erhältliche extrudierte Kupferchromit(III)-Katalysator "Cu-1230E 1/8 in.".

Bevorzugte Katalysatoren sind ebenfalls die in EP 361 755 A2 (Mitsui Petrochem. Ind.), Spalte 6, zur Acetonhydrierung gelehrten Katalysatoren.

Bevorzugt wird die Umsetzung bei einem Absolutdruck im Bereich von 10 bis 100 bar, besonders 30 bis 90 bar, weiter besonders 40 bis 80 bar, durchgeführt.

Die Umsetzung erfolgt bevorzugt bei einer Temperatur im Bereich von 40 bis 170 °C, besonders 50 bis 160 °C. Die Temperatur liegt bei kontinuierlicher Fahrweise weiter besonders im Bereich von 50 bis 80 °C am Reaktoreingang und 120 bis 150 °C am Reaktorausgang.

Die Katalysatorbelastung liegt bevorzugt im Bereich von 0,1 bis 0,7 kg/l•h, besonders 0,15 bis 0,6 kg/l•h, weiter besonders 0,2 bis 0,5 kg/l•h [kg Aceton / (Liter Katalysator • Stunde)].
(Liter Katalysator = Katalysatorschüttvolumen)

Die eingesetzte Wasserstoffmenge liegt bevorzugt im Bereich von 50 bis 350 Nl/l•h, besonders 100 bis 250 Nl/l•h, weiter besonders 120 bis 200 Nl/l•h, ganz besonders 150 bis 180 Nl/l•h
[Normliter / (Liter Katalysator • Stunde)]
(Nl = Normliter = auf Normalbedingungen (20 °C, 1 bar absolut) umgerechnetes Volumen).

Bevorzugt wird die Umsetzung bei einem Molverhältnis H₂ : Aceton im Bereich von 1,0 bis 3,0 mol/mol, besonders 1,0 bis 1,5 mol/mol, weiter besonders 1,0 bis 1,2 mol/mol, durchgeführt.

Das Verfahren lässt sich diskontinuierlich oder bevorzugt kontinuierlich wie folgt durchführen, wobei der Katalysator bevorzugt als Festbett im Reaktor angeordnet ist.

Die Umsetzung wird bevorzugt in einem Rohrreaktor oder Rohrbündelreaktor durchgeführt.

Die Hydrierung kann in der Flüssigphase oder in der Gasphase durchgeführt werden.

Sowohl beim Arbeiten in der Flüssigphase als auch beim Arbeiten in der Gasphase ist die Anwendung höherer Temperaturen und höherer Gesamtdrücke möglich. Der Druck im Reaktionsgefäß, welcher sich aus der Summe der Partialdrücke des Acetons und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Der Reaktionsaustrag der Aceton-Hydrierung wird bevorzugt direkt, also ohne weiteren Aufarbeitungsschritt wie Reinigung/Destillation, in die Aminierung zur MIPA-Herstellung eingesetzt.

Die Reaktion wird besonders bevorzugt in der Rieselphase in einem Schachtreaktor betrieben. Dabei werden sowohl die Flüssigkeit (Aceton + Rück-Iso-Propanol), als auch das Gas (Wasserstoff) von oben in den Reaktor eingespeist. Beim Durchgang durch den Reaktor ergibt sich aufgrund der Exothermie der Reaktion ein adiabater Temperaturanstieg, der aufgrund der Verdünnung des Acetons durch den Iso-Propanol-Kreislauf auf 30 bis 100 °C begrenzt wird. Nach dem Reaktorausgang durchläuft das Reaktionsgemisch einen Kühler, bevor in einem Abscheider die Gas- und die Flüssigphase getrennt werden. Ein Teil der Flüssigphase, z.B. < 50 Gew.-%, wird aufgereinigt oder bevorzugt direkt (unter Druck) in die Aminierung (zu MIPA) eingespeist. Der andere Teil der Flüssigphase wird bevorzugt als Rück-Iso-Propanol in die Hydrierung rückgeführt.

Alle Druckangaben beziehen sich auf den Absolutdruck.
Alle ppm-Angaben beziehen sich auf das Gewicht.

### Beispiele

### Katalysator ,A'

Der Katalysator ,A', ein Cu//Ni/Mo/ZrO₂ - Katalysator, wie in EP 696 572 A1 (BASF AG), siehe dort Beispiel 1, offenbart, wurde durch Fällung, Filtrierung, Temperung und Tablettierung (6 x 3 mm Tabletten) hergestellt.
Der Katalysator wies die folgende Zusammensetzung vor seiner Behandlung (Aktivierung) mit Wasserstoff auf:
50 Gew.-% NiO, 17 Gew.-% CuO und 1,5 Gew.-% MoO₃ auf ZrO₂.

### Beispiel 1

### Sequentielle Aminierung von Bio-Ethanol, Aceton, Bio-Ethanol und Iso-Propanol an einem Ni- und Cu-haltigen Katalysator

### a) Fahrweise mit Bio-Ethanol

In einen vertikal angeordneten Festbettreaktor wurden 800 ml (1313 g) des tablettenförmigen Ni/Cu-Katalysators 'A' eingefüllt. Nach Aktivierung mit Wasserstoff (250 °C, drucklos, 24 h) wurden von oben nach unten im geraden Durchgang 120 g/h biologisch hergestelltes Ethanol (Bio-Ethanol), welches zusätzlich geringe Anteile Ethylamine und Wasser enthielt (Zusammensetzung in Gew.-%: 87 % Ethanol, 5,7 % Ethylamine, 7 % Wasser, 1 ppm Schwefel, höhere Alkohole in Summe < 0,3 %) - entsprechend einer Katalysatorbelastung von 0,15 kg/(l•h) - und 57 g/h Ammoniak sowie 185 Nl/h Wasserstoff durch das Katalysatorbett geleitet. (Nl = Normliter = auf Normalbedingungen (20 °C, 1 bar) umgerechnetes Volumen). Der Druck betrug dabei 66 bar, die Temperatur lag bei 185 - 188 °C. Es wurden Bypass-Hochdruckproben vom Reaktionsgemisch nach dem Reaktor genommen (100 ml Druckzylinder) und im Labor in 40 ml Wasser entspannt. Die wässrigen Lösungen wurden gaschromatographisch analysiert: Säule RTX-5 Amin (I = 30 m; ID = 0,32 mm; Filmdicke (df) = 1,5 µm); 40 °C (10 min); auf 280 °C mit 10 °C/min; 280 °C (5 min); Trägergas He; Detektor FID. Die Ergebnisse sind als Gewichtsprozente des organischen Anteils angegeben. Der Versuch wurde für ca. 2900 h betrieben und es wurden dabei 91 - 94 % Ethylamine und 6 - 9 % nicht umgesetztes Ethanol erhalten.
Während dieser Laufzeit war der Katalysator mit insgesamt 356 kg Bioethanol mit einem ungefähren Schwefelgehalt von 1 ppm, d.h. mit 356 mg, entsprechend 271 Gew.-ppm auf dem Katalysator, Schwefel belastet worden.

### b) Fahrweise mit Aceton

Anschließend wurden ca. 180 h lang 128 g/h Aceton (Belastung 0,16 kg/(l•h)), 109 g/h Ammoniak sowie Wasserstoff in den Reaktor gefahren (Druck 40 bar).
Es wurden Bypass-Hochdruckproben vom Reaktionsgemisch nach dem Reaktor genommen (100 ml Druckzylinder) und im Labor in 40 ml Wasser entspannt. Die wässrigen Lösungen wurden gaschromatographisch analysiert: Säule DB1 (I = 30 m; ID = 0,32 mm; Filmdicke (df) = 3 µm); 50 °C (10 min); auf 280 °C mit 10 °C/min; 280 °C (17 min); Trägergas He; Detektor FID. Die Ergebnisse sind in der folgenden Tabelle als Gewichtsprozente angegeben:

| Laufzeit | Temp. | H2 | MIPA | iso-Propanol | DIPA | Summe C6-Amine |
|---|---|---|---|---|---|---|
| [h] | [°C] | [Nl/h] | [%] | [%] | [%] | [%] |
| 24 | 110 | 180 | **83,79** | 3,23 | 0,99 | 6,15 |
| 36 | 120 | 180 | **86,38** | 3,52 | 1,45 | 5,83 |
| 48 | 120 | 180 | **87,11** | 3,45 | 1,47 | 5,46 |
| 60 | 130 | 180 | **85,70** | 4,03 | 2,33 | 5,07 |
| 84 | 130 | 180 | **85,76** | 4,09 | 2,43 | 4,57 |
| 108 | 130 | 300 | **82,07** | 6,01 | 4,87 | 2,82 |
| 120 | 130 | 300 | **83,42** | 5,19 | 4,81 | 2,70 |
| 132 | 130 | 300 | **82,69** | 4,52 | 4,68 | 2,50 |
| 156 | 120 | 300 | **85,73** | 4,26 | 3,81 | 3,28 |
| 168 | 110 | 300 | **87,69** | 3,94 | 2,03 | 3,74 |
| 180 | 110 | 300 | **85,47** | 4,44 | 1,77 | 4,51 |

Aceton wurde über die Laufzeit vollständig umgesetzt.

Es ist ersichtlich, dass Aceton an einem durch vorherigen Betrieb mit Bioethanol verschwefelten Katalysator nur in schlechten Ausbeuten zu MIPA aminiert werden kann und insbesondere Kondensationsprodukte (C6-Amine) in beträchtlicher Menge gebildet werden.

### c) Fahrweise mit Bio-Ethanol (erfindungsgemäß)

Im Anschluss daran wurde der Katalysator ca. 500 h lang erneut mit Bio-Ethanol (analoge Bedingungen wie oben) betrieben.
Nach dieser Zeit waren insgesamt somit 414 kg Bio-Ethanol mit einem Schwefelgehalt von 1 ppm durch das Katalysatorbett gefahren worden, d.h. auf dem Katalysator waren unter Annahme vollständiger Adsorption 414 mg, entsprechend 315 Gew.-ppm, Schwefel vorhanden.

### d) Fahrweise mit Iso-Propanol

Abschließend wurden in diesem Experiment ca. 240 h lang Iso-Propanol (Belastungen 0,13 - 0,16 kg/(l•h)), 109 g/h Ammoniak (NH₃:Iso-Propanol-Molverhältnis = 3,0 bis 3,8) sowie 120 Nl/h Wasserstoff in den Reaktor gefahren (Druck 45 bar), vgl. Tabelle.
Es wurden Bypass-Hochdruckproben vom Reaktionsgemisch nach dem Reaktor genommen (100 ml Druckzylinder) und im Labor in 40 ml Wasser entspannt. Die wässrigen Lösungen wurden wie oben unter b) beschrieben gaschromatographisch analysiert. Die Ergebnisse sind in der folgenden Tabelle als Gewichtsprozente angegeben:

| Laufzeit | Temp. | iso-Propanol (Zulauf) | Belastung | MIPA | iso-Propanol | DIPA | Summe C6-Amine | U (iso-Propanol) | S (MIPA) |
|---|---|---|---|---|---|---|---|---|---|
| [h] | [°C] | [g/h] | [kg/(l•h)] | [%] | [%] | [%] | [%] | [%] | [%] |
| 24 | 180 | 128 | 0,16 | **88,16** | 6,82 | 2,39 | 0,00 | 93,18 | 94,61 |
| 36 | 180 | 128 | 0,16 | **89,65** | 6,40 | 3,14 | 0,00 | 93,60 | 95,77 |
| 48 | 180 | 128 | 0,16 | **89,94** | 6,24 | 3,36 | 0,00 | 93,76 | 95,93 |
| 60 | 180 | 128 | 0,16 | **89,68** | 6,95 | 2,91 | 0,00 | 93,05 | 96,38 |
| 72 | 185 | 128 | 0,16 | **87,72** | 5,97 | 5,81 | 0,00 | 94,03 | 93,28 |
| 84 | 185 | 128 | 0,16 | **87,84** | 5,93 | 5,80 | 0,00 | 94,07 | 93,38 |
| 108 | 180 | 128 | 0,16 | **89,20** | 7,04 | 3,36 | 0,00 | 92,96 | 95,96 |
| 132 | 180 | 128 | 0,16 | **90,23** | 6,25 | 3,20 | 0,00 | 93,75 | 96,25 |
| 156 | 180 | 128 | 0,16 | **90,02** | 6,40 | 3,23 | 0,00 | 93,60 | 96,17 |
| 168 | 180 | 128 | 0,16 | **89,56** | 6,81 | 3,28 | 0,00 | 93,19 | 96,11 |
| 180 | 180 | 100 | 0,13 | **91,88** | 4,23 | 3,51 | 0,00 | 95,77 | 95,93 |
| 192 | 180 | 100 | 0,13 | **91,35** | 4,77 | 3,37 | 0,00 | 95,23 | 95,93 |
| 204 | 180 | 100 | 0,13 | **91,22** | 4,77 | 3,61 | 0,00 | 95,23 | 95,79 |
| 216 | 180 | 100 | 0,13 | **90,60** | 5,54 | 3,47 | 0,00 | 94,46 | 95,91 |
| 228 | 180 | 100 | 0,13 | **91,35** | 5,08 | 3,20 | 0,00 | 94,92 | 96,24 |
| 240 | 180 | 100 | 0,13 | **91,12** | 5,07 | 3,45 | 0,00 | 94,93 | 95,99 |
| 252 | 180 | 100 | 0,13 | **91,40** | 4,81 | 3,43 | 0,00 | 95,19 | 96,02 |
| 264 | 180 | 100 | 0,13 | **91,64** | 4,55 | 3,41 | 0,01 | 95,46 | 96,00 |

Es ist ersichtlich, dass sich durch Aminierung von Iso-Propanol wesentlich bessere MIPA-Ausbeuten erzielen lassen als durch Aminierung von Aceton und insbesondere keine Kondensationsprodukte (C6-Amine) entstehen. Der Umsatz in % ist hier definiert als "100 % - Iso-Propanol [Gew.-%]". Die Selektivität in % ist hier definiert als "Quotient aus MIPA [Gew.-%] und Umsatz [%], multipliziert mit 100".

### Beispiel 2 (Vergleichsbeispiel):

Aminierung von Iso-Propanol zu MIPA an einem frischen Katalysator, der vorher nicht für die Aminierung von Bio-Ethanol eingesetzt wurde

In einem vertikal angeordneten Festbettreaktor wurden 130 ml des tablettenförmigen Ni/Cu-Katalysators ,A' eingefüllt. Nach Aktivierung mit Wasserstoff wurden im geraden Durchgang 19 g/h Iso-Propanol - entsprechend einer Katalysatorbelastung von 0,15 kg/(l•h) - und 18 g/h Ammoniak (NH₃: Iso-Propanol-Molverhältnis = 3,3) sowie 29 Nl/h Wasserstoff durch das Katalysatorbett geleitet. Der Druck betrug dabei 45 bar, die Temperatur lag bei 180 °C. Während einer Laufzeit von ca. 90 h wurden im Mittel folgende Werte erhalten: 89 % MIPA; 6,1 % Iso-Propanol; 5,1 % Di-(iso-Propyl)-amin sowie 0,00 % Kondensationsprodukte (C6-Amine); (GC-Methode wie unter b) beschrieben). Gemäß der Definition in Beispiel 1d) betrug der Iso-Propanol-Umsatz 93,9 % und die MIPA-Selektivität 94,8 %.

Somit ist ersichtlich, dass die Aminierung von Iso-Propanol zu MIPA an einem vorher für die Aminierung von schwefelhaltigem Bio-Ethanol eingesetzten Katalysator zu mindestens ebenso guten Resultaten bezüglich Umsatz und Selektivität führt wie die Aminierung von Iso-Propanol zu MIPA an einem frischen Katalysator.

### Beispiel 3

### Sequentielle Aminierung von Bio-Ethanol und Iso-Propanol an einem Kobalt-haltigen Katalysator

### a) Fahrweise mit Bio-Ethanol

In einem vertikal angeordneten Festbettreaktor wurden 800 ml (773 g) des kommerziell bei der Firma Süd-Chemie erhältlichen Katalysators G-62RS (siehe "General Catalogue Süd-Chemie Catalysts" (2007)) eingefüllt. Nach Aktivierung mit Wasserstoff bei bis zu 240 °C und 40 bar (ca. 24 h) wurden von oben nach unten im geraden Durchgang 120 - 200 g/h biologisch hergestelltes Ethanol, welches zusätzlich geringe Anteile Ethylamine und Wasser enthielt (Zusammensetzung: 89 % Ethanol, 3,7 % Ethylamine, 7 % Wasser, 1 ppm Schwefel, höhere Alkohole in Summe < 0,3 %) - entsprechend einer Katalysatorbelastung von 0,15 - 0,25 kg/(l•h) - sowie 57 - 96 g/h Ammoniak sowie zwischen 130 und 275 Nl/h Wasserstoff durch das Katalysatorbett geleitet. Der Druck betrug dabei 66 bar, die Temperatur lag bei 175 - 194 °C. Es wurden Bypass-Hochdruckproben vom Reaktionsgemisch nach dem Reaktor genommen (100 ml Druckzylinder) und im Labor in 40 ml Wasser entspannt. Die wässrigen Lösungen wurden gaschromatographisch analysiert: Säule RTX-5 Amin (I = 30 m; ID = 0.32 mm; Filmdicke (df) = 1.5 µm); 40 °C (10 min); auf 280 °C mit 10 °C/min; 280 °C (5 min); Trägergas He; Detektor FID. Die Ergebnisse sind als Gewichtsprozente des organischen Anteils angegeben. Der Versuch wurde für ca. 2500 h betrieben und es wurden dabei 85 - 96 % Ethylamine und 3 - 14 % nicht umgesetztes Ethanol erhalten.
Während dieser Laufzeit war der Katalysator mit insgesamt 347 kg Bio-Ethanol mit einem ungefähren Schwefelgehalt von 1 ppm, d.h. mit 347 mg Schwefel belastet worden (entsprechend 449 Gew.-ppm).

### b) Fahrweise mit Iso-Propanol

Anschließend wurden in diesem Experiment ca. 580 h lang Iso-Propanol (Belastungen 0,13 - 0,17 kg/(l•h)), 93 - 118 g/h Ammoniak (NH₃:Iso-Propanol-Molverhältnis = 2,8 bis 3,9) sowie 120 - 126 Nl/h Wasserstoff in den Reaktor gefahren (Druck 45 bar), vgl. Tabelle.
Es wurden Bypass-Hochdruckproben vom Reaktionsgemisch nach dem Reaktor genommen (100 ml Druckzylinder) und im Labor in 40 ml Wasser entspannt. Die wässrigen Lösungen wurden wie oben unter Beispiel 1 b beschrieben gaschromatographisch analysiert. Die Ergebnisse sind in der folgenden Tabelle als Gewichtsprozente angegeben:

| Laufzeit | Temp. | iso-Propanol (Zulauf) | NH3 | MIPA | iso-Propanol | DIPA | Summe C6-Amine | U (iso-Propanol) | S (MIPA) |
|---|---|---|---|---|---|---|---|---|---|
| [h] | [°C] | [g/h] | [g/h] | [%] | [%] | [%] | [%] | [%] | [%] |
| 48 | 180 | 128 | 109 | **85,11** | 11,99 | 2,19 | 0,00 | 88,02 | 96,70 |
| 72 | 180 | 128 | 109 | **84,44** | 11,88 | 2,21 | 0,00 | 88,13 | 95,82 |
| 96 | 185 | 128 | 109 | **86,04** | 9,38 | 3,57 | 0,00 | 90,62 | 94,95 |
| 120 | 185 | 128 | 109 | **86,18** | 9,85 | 3,57 | 0,00 | 90,15 | 95,59 |
| 144 | 188 | 123 | 118 | **86,51** | 9,59 | 3,58 | 0,00 | 90,41 | 95,69 |
| 168 | 188 | 126 | 109 | **86,41** | 8,77 | 4,47 | 0,00 | 91,23 | 94,72 |
| 216 | 193 | 130 | 93 | **84,69** | 7,83 | 7,10 | 0,00 | 92,17 | 91,89 |
| 240 | 193 | 132 | 109 | **84,80** | 7,94 | 6,88 | 0,00 | 92,06 | 92,12 |
| 264 | 193 | 132 | 105 | **84,37** | 7,78 | 7,41 | 0,00 | 92,22 | 91,49 |
| 288 | 193 | 133 | 105 | **85,37** | 8,18 | 6,09 | 0,00 | 91,82 | 92,97 |
| 312 | 193 | 130 | 111 | **83,96** | 8,54 | 7,07 | 0,00 | 91,46 | 91,80 |
| 336 | 193 | 100 | 107 | **87,23** | 5,78 | 6,57 | 0,00 | 94,22 | 92,58 |
| 360 | 193 | 100 | 108 | **86,87** | 5,78 | 6,31 | 0,00 | 94,22 | 92,19 |
| 432 | 193 | 100 | 109 | **85,14** | 5,21 | 8,04 | 0,00 | 94,79 | 89,82 |
| 456 | 193 | 100 | 109 | **85,66** | 5,18 | 7,76 | 0,00 | 94,82 | 90,34 |
| 480 | 185 | 100 | 109 | **88,81** | 6,61 | 3,77 | 0,00 | 93,39 | 95,10 |
| 552 | 185 | 100 | 109 | **88,97** | 6,38 | 4,01 | 0,00 | 93,62 | 95,03 |
| 576 | 185 | 100 | 109 | **88,50** | 6,61 | 3,97 | 0,01 | 93,39 | 94,76 |

Es ist ersichtlich, dass die Aminierung von Iso-Propanol zu MIPA in hohen Selektivitäten und insbesondere unter Vermeidung von Kondensationsprodukten (C6-Amine) gelingt, obwohl sie an einem Katalysator durchgeführt wurde, der vorher für die Aminierung von Schwefel-haltigem Bio-Ethanol verwendet wurde und somit Schwefel-kontaminiert war. Der Umsatz in % ist hier definiert als "100 % - Iso-Propanol [Gew.-%]". Die Selektivität in % ist hier definiert als "Quotient aus MIPA [Gew.-%] und Umsatz [%], multipliziert mit 100".

### Methoden der Schwefelbestimmung in Bio-Ethanol

- Die Methode der Coulometrie ist beschrieben in der DIN 51400-7.
- Die Probenvorbereitung für die Verbrennung nach Wickbold beschreibt z.B. die DIN 51408-1, auch nachzulesen bei "Quantitative organische Elementaranalyse", Friedrich Ehrenberger, ISBN 3-527-28056-1, Seite 424 ff. Die Messung des bei der Verbrennung entstandenen und absorbierten Sulfats erfolgt mittels Ionenchromatografie. Zugehörende Literatur ist z.B. "Ionenchromatographie", Joachim Weiß, ISBN 3-327-28702-7, Seite 68 ff.

## Patentansprüche

1. Verfahren zur Herstellung von Ethylaminen und Mono-iso-Propylamin (MIPA), **dadurch gekennzeichnet, dass** man Bio-Ethanol mit Ammoniak in Gegenwart von Wasserstoff und eines Heterogen-Katalysators zu Ethylaminen umsetzt, wobei das Bio-Ethanol einen Gehalt an Schwefel und/oder schwefelhaltigen Verbindungen von ≥ 0,1 Gew.-ppm bis 10 Gew.-ppm (berechnet S) aufweist, und danach in Gegenwart desselben Katalysators IsoPropanol mit Ammoniak in Gegenwart von Wasserstoff zu MIPA umsetzt, wobei zwischen den beiden Umsetzungen keine Katalysatoraktivierung durch S-Entfernung (Entgiftung) erfolgt.

2. Verfahren nach Anspruch 1, **dadurch gekennzeichnet, dass** man die Umsetzung von Bio-Ethanol mit Ammoniak über einen Zeitraum durchführt, an dessen Ende der S-Gehalt des Katalysators < 700 Gew.-ppm beträgt.

3. Verfahren nach Anspruch 1 oder 2, **dadurch gekennzeichnet, dass** man die Umsetzungen in Gegenwart eines kupfer- und/oder nickel- und/oder kobalthaltigen Heterogen-Katalysators durchführt.

4. Verfahren nach einem der vorhergehenden Ansprüche, **dadurch gekennzeichnet, dass** man die Umsetzungen in Gegenwart eines Heterogen-Katalysators durchführt, dessen Gehalt an Nickel mehr als 90 Gew.-%, bezogen auf alle gegebenenfalls enthaltenen Metalle der Gruppe VIII des Periodensystems, beträgt.

5. Verfahren nach einem der vorhergehenden Ansprüche 1 bis 3, **dadurch gekennzeichnet, dass** man die Umsetzungen in Gegenwart eines Heterogen-Katalysators durchführt, dessen Gehalt an Kobalt mehr als 90 Gew.-%, bezogen auf alle gegebenenfalls enthaltenen Metalle der Gruppe VIII des Periodensystems, beträgt.

6. Verfahren nach einem der vorhergehenden Ansprüche, **dadurch gekennzeichnet, dass** man die Umsetzungen in Gegenwart eines kupfer- und nickelhaltigen Heterogen-Katalysators durchführt.

7. Verfahren nach einem der vorhergehenden Ansprüche, **dadurch gekennzeichnet, dass** man die Umsetzungen in Gegenwart eines kupfer- und nickel- und kobalthaltigen Heterogen-Katalysators durchführt.

8. Verfahren nach einem der vorhergehenden Ansprüche, **dadurch gekennzeichnet, dass** der Heterogen-Katalysator Aluminiumoxid, Siliziumdioxid, Titandioxid und/oder Zirkoniumdioxid als Trägermaterial enthält.

9. Verfahren nach einem der vorhergehenden Ansprüche, **dadurch gekennzeichnet, dass** der Heterogen-Katalysator in seiner katalytisch aktiven Masse vor der Behandlung mit Wasserstoff
20 bis 85 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
14 bis 70 Gew.-% sauerstoffhaltige Verbindungen des Nickels, berechnet als NiO, und 0 bis 5 Gew.-% sauerstoffhaltige Verbindungen des Molybdäns, berechnet als MoO₃, enthält.

10. Verfahren nach einem der vorhergehenden Ansprüche, **dadurch gekennzeichnet, dass** man die Umsetzungen jeweils bei einem Absolutdruck im Bereich von 10 bis 100 bar durchführt.

11. Verfahren nach einem der vorhergehenden Ansprüche, **dadurch gekennzeichnet, dass** man die Umsetzungen jeweils bei einer Temperatur im Bereich von 130 bis 230 °C durchführt.

12. Verfahren nach einem der vorhergehenden Ansprüche, **dadurch gekennzeichnet, dass** man die Umsetzungen jeweils kontinuierlich durchführt.

13. Verfahren nach einem der vorhergehenden Ansprüche, **dadurch gekennzeichnet, dass** zwischen den beiden Umsetzungen keine chemische Katalysatorbehandlung erfolgt.

14. Verfahren nach einem der vorhergehenden Ansprüche, **dadurch gekennzeichnet, dass** man die Umsetzungen jeweils bei einer Katalysatorbelastung im Bereich von 0,05 bis 0,35 kg Alkohol / (Liter Katalysator • Stunde) durchführt.

15. Verfahren nach einem der vorhergehenden Ansprüche, **dadurch gekennzeichnet, dass** die eingesetzte Wasserstoffmenge jeweils im Bereich von 50 bis 350 Normliter / (Liter Katalysator • Stunde) liegt.

16. Verfahren nach einem der vorhergehenden Ansprüche, **dadurch gekennzeichnet, dass** das Molverhältnis NH₃ : Ethanol im Bereich von 0,4 bis 10 liegt.

17. Verfahren nach einem der vorhergehenden Ansprüche, **dadurch gekennzeichnet, dass** das Molverhältnis NH₃ : Iso-Prepanol im Bereich von 1,0 bis 10 liegt.

18. Verfahren nach einem der vorhergehenden Ansprüche, **dadurch gekennzeichnet dass** man Iso-Propanol einsetzt, welches zuvor durch Hydrierung von Aceton in Gegenwart eines kupfer- und/oder nickel- und/oder kobalthaltigen Katalysators hergestellt wurde.

19. Verfahren nach dem vorhergehenden Anspruch, **dadurch gekennzeichnet, dass** der Reaktionsaustrag der Aceton-Hydrierung direkt in die Aminierung zur MIPA-Herstellung eingesetzt wird.

20. Verfahren nach einem der vorhergehenden Ansprüche, **dadurch gekennzeichnet, dass** man die Umsetzungen jeweils in der Gasphase durchführt.

21. Verfahren nach einem der vorhergehenden Ansprüche, **dadurch gekennzeichnet, dass** man die Umsetzungen jeweils in einem Rohrreaktor oder Rohrbündelreaktor durchführt.

22. Verfahren nach einem der vorhergehenden Ansprüche, **dadurch gekennzeichnet, dass** der Katalysator als Festbett angeordnet ist.

23. Verfahren nach einem der vorhergehenden Ansprüche, **dadurch gekennzeichnet, dass** das Bio-Ethanol einen Gehalt an Schwefel und/oder schwefelhaltigen Verbindungen von ≥ 0.2 Gew.-ppm bis 10 Gew.-ppm (berechnet S) aufweist.

24. Verfahren nach einem der vorhergehenden Ansprüche, **dadurch gekennzeichnet, dass** man die Umsetzung von Bio-Ethanol mit Ammoniak über einen Zeitraum durchführt, an dessen Ende der S-Gehalt des Katalysators 150 bis < 600 Gew.-ppm beträgt.

## Claims

1. A process for preparing ethylamines and monoisopropylamine (MIPA), which comprises reacting bioethanol with ammonia in the presence of hydrogen and of a heterogeneous catalyst to give ethylamines, said bioethanol having a content of sulfur and/or sulfur compounds of ≥ 0.1 ppm by weight to 10 ppm by weight (calculated S), and then reacting isopropanol with ammonia in the presence of the same catalyst and in the presence of hydrogen to give MIPA, there being no catalyst activation by sulfur removal (poison removal) between the two reactions.

2. The process according to claim 1, wherein the sulfur content of the catalyst at the end of the period over which the reaction of bioethanol with ammonia is performed is < 700 ppm by weight.

3. The process according to claim 1 or 2, wherein the reactions are performed in the presence of a heterogeneous copper and/or nickel and/or cobalt catalyst.

4. The process according to any of the preceding claims, wherein the reactions are performed in the presence of a heterogeneous catalyst having a nickel content of more than 90% by weight, based on any and all metals of group VIII of the Periodic Table present.

5. The process according to any of the preceding claims 1 to 3, wherein the reactions are performed in the presence of a heterogeneous catalyst having a cobalt content of more than 90% by weight, based on any and all metals of group VIII of the Periodic Table present.

6. The process according to any of the preceding claims, wherein the reactions are performed in the presence of a heterogeneous copper and nickel catalyst.

7. The process according to any of the preceding claims, wherein the reactions are performed in the presence of a heterogeneous copper and nickel and cobalt catalyst.

8. The process according to any of the preceding claims, wherein the heterogeneous catalyst comprises aluminum oxide, silicon dioxide, titanium dioxide and/or zirconium dioxide as support material.

9. The process according to any of the preceding claims, wherein the heterogeneous catalyst comprises, in its catalytically active composition before the treatment with hydrogen,
20 to 85% by weight of oxygen compounds of zirconium, calculated as ZrO₂,
1 to 30% by weight of oxygen compounds of copper, calculated as CuO,
14 to 70% by weight of oxygen compounds of nickel, calculated as NiO, and
0 to 5% by weight of oxygen compounds of molybdenum, calculated as MoO₃.

10. The process according to any of the preceding claims, wherein the reactions are each performed at an absolute pressure in the range from 10 to 100 bar.

11. The process according to any of the preceding claims, wherein the reactions are each performed at a temperature in the range from 130 to 230°C.

12. The process according to any of the preceding claims, wherein the reactions are each performed continuously.

13. The process according to any of the preceding claims, wherein there is no chemical catalyst treatment between the two reactions.

14. The process according to any of the preceding claims, wherein the reactions are each performed at a catalyst space velocity in the range from 0.05 to 0.35 kg of alcohol / (liter of catalyst • hour).

15. The process according to any of the preceding claims, wherein the amount of hydrogen used is in each case in the range from 50 to 350 standard liters / (liter of catalyst • hour).

16. The process according to any of the preceding claims, wherein the molar NH₃ : ethanol ratio is in the range from 0.4 to 10.

17. The process according to any of the preceding claims, wherein the molar NH₃ : isopropanol ratio is in the range from 1.0 to 10.

18. The process according to any of the preceding claims, wherein isopropanol which has been prepared beforehand by hydrogenation of acetone in the presence of a copper and/or nickel and/or cobalt catalyst is used.

19. The process according to the preceding claim, wherein the reaction output of the acetone hydrogenation is used directly in the amination for MIPA preparation.

20. The process according to any of the preceding claims, wherein the reactions are each performed in the gas phase.

21. The process according to any of the preceding claims, wherein the reactions are each performed in a tubular reactor or a shell and tube reactor.

22. The process according to any of the preceding claims, wherein the catalyst is arranged as a fixed bed.

23. The process according to any of the preceding claims, wherein the bioethanol has a content of sulfur and/or sulfur compounds of ≥ 0.2 ppm by weight to 10 ppm by weight (calculated S).

24. The process according to any of the preceding claims, wherein the sulfur content of the catalyst at the end of the period over which the reaction of bioethanol with ammonia is performed is 150 to < 600 ppm by weight.

## Revendications

1. Procédé pour la préparation d'éthylamines et de mono-isopropylamine (MIPA), **caractérisé en ce qu'**on transforme du bioéthanol avec de l'ammoniac en présence d'hydrogène et d'un catalyseur hétérogène en éthylamines, le bioéthanol présentant une teneur en soufre et/ou en composés soufrés ≥ 0,1 ppm en poids à 10 ppm en poids (calculée sous forme S) et on transforme ensuite, en présence du même catalyseur, de l'isopropanol avec de l'ammoniac en présence d'hydrogène en MIPA, aucune activation du catalyseur par élimination de S (décontamination) n'étant réalisée entre les deux transformations.

2. Procédé selon la revendication 1, **caractérisé en ce qu'**on réalise la transformation du bioéthanol avec l'ammoniac sur un laps de temps à la fin duquel la teneur en S du catalyseur est < 700 ppm en poids.

3. Procédé selon la revendication 1 ou 2, **caractérisé en ce qu'**on réalise les transformations en présence d'un catalyseur hétérogène contenant du cuivre et/ou du nickel et/ou du cobalt.

4. Procédé selon l'une quelconque des revendications précédentes, **caractérisé en ce qu'**on réalise les transformations en présence d'un catalyseur hétérogène, dont la teneur en nickel est supérieure à 90%, par rapport à tous les métaux du groupe VIII du système périodique le cas échéant contenus.

5. Procédé selon l'une quelconque des revendications précédentes 1 à 3, **caractérisé en ce qu'**on réalise les transformations en présence d'un catalyseur hétérogène, dont la teneur en cobalt est supérieure à 90%, par rapport à tous les métaux du groupe VIII du système périodique le cas échéant contenus.

6. Procédé selon l'une quelconque des revendications précédentes, **caractérisé en ce qu'**on réalise les transformations en présence d'un catalyseur hétérogène contenant du cuivre et du nickel.

7. Procédé selon l'une quelconque des revendications précédentes, **caractérisé en ce qu'**on réalise les transformations en présence d'un catalyseur hétérogène contenant du cuivre et du nickel et du cobalt.

8. Procédé selon l'une quelconque des revendications précédentes, **caractérisé en ce que** le catalyseur hétérogène contient de l'oxyde d'aluminium, du dioxyde de silicium, du dioxyde de titane et/ou du dioxyde de zirconium comme matériau support.

9. Procédé selon l'une quelconque des revendications précédentes, **caractérisé en ce que** le catalyseur hétérogène contient, dans sa masse catalytiquement active, avant le traitement avec l'hydrogène
20 à 85% en poids de composés oxygénés du zirconium, calculés sous forme de ZrO₂,
1 à 30% en poids de composés oxygénés du cuivre, calculés sous forme de CuO,
14 à 70% en poids de composés oxygénés du nickel, calculés sous forme de NiO, et
0 à 5% en poids de composés oxygénés du molybdène, calculés sous forme de MoO₃.

10. Procédé selon l'une quelconque des revendications précédentes, **caractérisé en ce qu'**on réalise les transformations à chaque fois à une pression absolue dans la plage de 10 à 100 bars.

11. Procédé selon l'une quelconque des revendications précédentes, **caractérisé en ce qu'**on réalise les transformations à chaque fois à une température dans la plage de 130 à 230°C.

12. Procédé selon l'une quelconque des revendications précédentes, **caractérisé en ce qu'**on réalise les transformations à chaque fois en continu.

13. Procédé selon l'une quelconque des revendications précédentes, **caractérisé en ce qu'**aucun traitement chimique du catalyseur n'a lieu entre les deux transformations.

14. Procédé selon l'une quelconque des revendications précédentes, **caractérisé en ce qu'**on réalise les transformations à chaque fois à une charge du catalyseur dans la plage de 0,05 à 0,35 kg d'alcool/(litre de catalyseur.heure).

15. Procédé selon l'une quelconque des revendications précédentes, **caractérisé en ce que** la quantité d'hydrogène utilisée se situe à chaque fois dans la plage de 50 à 350 litres normaux/(litre de catalyseur.heure).

16. Procédé selon l'une quelconque des revendications précédentes, **caractérisé en ce que** le rapport molaire NH₃:éthanol se situe dans la plage de 0,4 à 10.

17. Procédé selon l'une quelconque des revendications précédentes, **caractérisé en ce que** le rapport molaire NH₃:isopropanol se situe dans la plage de 1,0 à 10.

18. Procédé selon l'une quelconque des revendications précédentes, **caractérisé en ce qu'**on utilise de l'isopropanol qui a été préparé au préalable par hydrogénation d'acétone en présence d'un catalyseur contenant du cuivre et/ou du nickel et/ou du cobalt.

19. Procédé selon la revendication précédente, **caractérisé en ce que** le produit de réaction de l'hydrogénation de l'acétone est utilisé directement dans l'amination pour la préparation de MIPA.

20. Procédé selon l'une quelconque des revendications précédentes, **caractérisé en ce qu'**on réalise les transformations à chaque fois en phase gazeuse.

21. Procédé selon l'une quelconque des revendications précédentes, **caractérisé en ce qu'**on réalise les transformations à chaque fois dans un réacteur tubulaire ou dans un réacteur à faisceau tubulaire.

22. Procédé selon l'une quelconque des revendications précédentes, **caractérisé en ce que** le catalyseur est disposé sous forme de lit fixe.

23. Procédé selon l'une quelconque des revendications précédentes, caractérisé en ce le bioéthanol présente une teneur en soufre et/ou en composés soufrés ≥ 0,2 ppm en poids à 10 ppm en poids (calculée sous forme S).

24. Procédé selon l'une quelconque des revendications précédentes, **caractérisé en ce qu'**on réalise la transformation du bioéthanol avec l'ammoniac sur un laps de temps à la fin duquel la teneur en S du catalyseur est de 150 à < 600 ppm en poids.
